## Europäisches Patentamt
## European Patent Office
## Office européen des brevets

(19)

(11) Publication number: **0 204 586**
**A2**

(12)

# EUROPEAN PATENT APPLICATION

(21) Application number: **86304354.3**

(22) Date of filing: **06.06.86**

(51) Int. Cl.⁴: **A 61 K 7/06**

(30) Priority: **07.06.85 JP 124609/85**

(43) Date of publication of application:
**10.12.86 Bulletin 86/50**

(84) Designated Contracting States:
**CH DE FR LI**

(71) Applicant: **SHIONOGI & CO., LTD.**
**12, Dosho-machi 3-chome Higashi-ku**
**Osaka 541(JP)**

(72) Inventor: **Ukita, Hisako**
**5-10-7, Uematsu-cho**
**Yao-shi Osaka(JP)**

(72) Inventor: **Kawai, Sadao**
**1-15-7, Kawai**
**Matsubara-shi Osaka(JP)**

(72) Inventor: **Egawa, Shohei**
**2-4-15, Nanatsumatsu-cho**
**Amagasaki-shi Hyogo(JP)**

(74) Representative: **Bizley, Richard Edward et al,**
**BOULT, WADE & TENNANT 27 Furnival Street**
**London EC4A 1PQ(GB)**

(54) Stabilizer for hair compositions and such compositions.

(57) The use of vegetable oils in hair lotions is achieved, without the need for much, or any, surfactant, by employing in a solubilizing function a branched $C_{12}$-$C_{22}$ saturated monocarboxylic acid, e.g. at 5-31 w/v %.

EP 0 204 586 A2

## SOLUBILIZER FOR HAIR COMPOSITIONS
## AND SUCH COMPOSITIONS

The present invention relates to hair lotions for making the hair emollient, lubricating the hair, and dressing up the hair smoothly and having the texture of vegetable oils.

Hair is often exposed to the danger of being injured by perming or dyeing. There are many complaints about available hair lotions, such as the feeling that the hair is controllable but is concurrently tough or sticky and vice versa. The hair lotions of this invention can alleviate dry scalp, make the hair smooth and manageable, and give it a good feel characteristic of vegetable oils.

Vegetable oils such as castor oil have been well used as active ingredients for hair preparations because they are excellent not only in controlling unmanageable hair with less irritation to the skin or the hair but also in the feel of the hair after application.

In order to solubilize vegetable oils such as castor oil in an aqueous alcohol when liquid preparations of the type used recently are required, in the prior art a large amount of surfactant has been used in solubilizing said vegetable oils in the aqueous alcohol.

Various means for solubilizing the oils, e.g. methods directly solubilizing after mixing castor oil and surfactant in an aqueous alcohol and methods using a castor oil derivative which is chemically bound with a surfactant, have been attempted. Practically the same amount of surfactant and of castor oil was necessary for solubilization whichever means was used. Such means are disclosed in JPN Unexam. Pat. Publication Nos. 59-62511 and 55-124711.

Generally speaking, the application of surfactants in large amounts causes unpleasant feelings such as dry scalp, irritation, itching, stiff hair, tough or sticky hair, and the like. Although many kinds of surfactants have been examined in order to reduce such unpleasant feelings after application of compositions containing them, hair grooming compositions giving a good feel have not been successfully developed yet because such unpleasant feelings are caused by the natural characteristics of surfactants.

This invention relates to the use as a solubilizer in a hair composition of a branched $C_{12}$-$C_{22}$ saturated monocarboxylic acid 5-31 w/v % and, if necessary, a surfactant 0-5 w/v %. Such a hair composition or lotion may include castor oil, a solubilizer, an alcohol, and water as essential components. In a specific class of embodiments, this invention provides hair lotions consisting essentially of the following components:

| | |
|---|---|
| castor oil | 5-22 w/v % |
| isostearic acid | 5-31 w/v % |
| ethanol | 85-38 v/v % |
| a surfactant | 0-5 w/v %, and |
| water | necessary amount to make 100%. |

In the accompanying drawings:-

Figure 1 shows the results of a feel test on compositions 7 (●) and 8 (◎) of this invention, and commercially available compositions A (△) and B (○).

Figure 2 shows the results of a feel test on compositions 7 (●), 8 (◎), and 10 (○) of this invention, and commercially available composition A (△).

The present invention has solved the aforementioned problem in the discovery that vegetable oils such as castor oil can be solubilized in aqueous alcohols if a branched $C_{12}-C_{22}$ saturated monocarboxylic acid, preferably isostearic acid, is used at a rate of 5-31 w/v %: this invention is based on this finding. Appropriate surfactants can also be added at a rate of 0-5 w/v %, if required.

In more detail, hair lotions of this invention can be prepared by using as essential components (a) castor oil 5-22 w/v %, (b) isostearic acid 5-31 w/v %, (c) ethanol 85-38 v/v %, (d) a surfactant 0-5 w/v %, and (e) a necessary amount of water to make 100%. As a matter of course, perfume or colour additives and, if required, other additives such as preservatives, pH adjusters, and the like may also be added.

Vegetable oils applicable to this invention include castor oil, olive oil, tsubaki oil (camellia oil), and the like, which may be used at a range of 5-22 w/v %. When used in amounts less than the aforesaid lower limit, no preferred vegetable oil feel is given nor is the hair satisfactorily controllable. Above the upper limit, the compositions can cause unpleasant feelings such as tough or sticky hair; in any event, the preferred compositions cannot be prepared outside the above range.

As branched $C_{12}$-$C_{22}$ saturated monocarboxylic acids, iso $C_{12}$-$C_{22}$ monocarboxylic acids such as isolauric acid, isomyristic acid, isopalmitic acid, isostearic acid, isoarachic acid, isobehenic acid and the like are exemplified. Among others, isostearic acid is most preferred in this invention. They may be used singly or in combination, e.g. at a rate of 5-31 w/v %, more preferably 7-20 w/v %, most preferably 8-15 w/v %, based upon the whole composition. The amount of the carboxylic acid varies with the amount of surfactant to be used: when no surfactant is used at all, the branched $C_{12}$-$C_{22}$ saturated monocarboxylic acid should be used in an amount equal to or more than the amount of vegetable oil used.

Appropriate surfactants are mainly nonionic-type surfactants in this invention and include polyethyleneglycol alkyl ethers, polyethyleneglycol fatty acid esters, polyoxyethylene (hereinafter abbreviated as POE) sorbitan fatty acid esters, POE alkylphenyl ethers, and POE castor oil or POE hydrogenated castor oil. More specifically, POE sorbitan monooleate, POE cetyl ether, POE lauryl ether, POE nonylphenyl ether, or the like, are preferred. They can be used singly or in combination at a rate of 0-5 w/v % based upon the whole composition.

Various kinds of additives can, if desired, be added to compositions of this invention, e.g. hair lotions. For example, film-forming substances, conditioning materials, colour additives, perfumes, preservatives, emollients, defoaming agents, hair-grooming agents, antidandruff agents, antioxidants, antihistamines, pH-adjusters or the like may be applied. Substances generally used in the cosmetic industry are preferably employed, especially recommended are those allowed by authorities under, e.g Raw Material Standards for Cosmetics or Drugs, Cosmetics and Medical Instruments Act.

Film-forming substances include cationic co-polymers, acrylic resin alkanolamine solution, and the like. Conditioning materials include oleyl alcohol, isopropyl myristate (IPM), isopropyl palmitate (IPP), hexyl laurate, octyldodecyl myristate, octyldodecanol, polyethylene glycol (hereinafter abbreviated as PEG; for example, PEG 200, PEG 300, PEG 400, PEG 600, and the like), butyl stearate, and the like. Emollients include glycerin, propylene glycol (hereinafter abbreviated as PG), sodium pyrrolidone carbocylate (hereinafter abbreviated as PCANa), and the like. Hair-grooming agents include vitamins (vitamin-$B_2$, -$B_5$, -D, and -E, pantothenol, biotin, and the like) and amino acids (valine, leucine, threonine, lysine, methionine, phenylalanine, tryptophan, and the like). pH-Adjusters include citric acid, lactic acid, sodium lactate, di- or tri-ethanolamine, sodium hydroxide, and the like. They may be added if desired.

Alcohols which are only low irritants should be employed and, especially, ethanol is recommended. The alcohols are added to the aforementioned components and a necessary amount of water is then added to make the whole 100%.

An example of the procedure for manufacturing hair lotions of this invention is illustrated below.

A weighted amount of vegetable oil (castor oil, tsubaki oil, olive oil, or the like) is added to isostearic acid or isopalmitic acid and the mixture is stirred well. A conditioning agent, and then ethanol are added to the mixture while being stirred. Additives such as perfume, antioxidant, preservative, colour additive and the like, are further added thereto and a necessary amount of water is added to make the whole 100 v/v %.

The following Examples and Experiments serve to illustrate the practical preparation and effect of compositions of this invention, but are not intended to limit the scope of this invention.

EXAMPLE 1

To a mixture of 12 g of castor oil and 10 g of isostearic acid are added 6 g of oleyl alcohol and then 59 ml of ethanol while stirring. Acrylic resin alkanolamine solution (2 g), 0.2 g of d-pantothenol, and 2.0 g of propylene glycol are added in order and the mixture is blended well. The mixture is adjusted to a pH of about 6.0 with 0.5 ml of 50% lactic acid and 2.0 ml of 50% sodium lactate, and finally a necessary amount of water is added thereto to make the whole 100 ml.

## EXAMPLES 2 - 10

In the same manner as shown above, the following hair lotions were prepared:

| Example No.<br>Components | 2 | 3 | 4 | 5 | 6 | 7 | 8 | 9 | 10 |
|---|---|---|---|---|---|---|---|---|---|
| d-Pantothenol | 0.2 | 0.2 | 0.2 | | | 0.2 | 0.2 | 0.2 | 0.2 |
| Castor oil | 10 | 10 | 10 | 5 | 22 | 8 | 10 | 8 | 8 |
| Isostearic acid | 10 | 10 | 10 | 5 | 31 | 10 | 10 | 11.8 | 10 |
| Oleyl alcohol | 6 | 6 | 6 | | | 6 | 6 | 6 | 6 |
| Polyethylene Glycol 300 | | | | 1 | 1 | | | | |
| Cationic co-polymer | | 2 | 2 | | | | | 2 | 2 |
| Acrylic Resin Alkanolamine soln. | 2 | | | | | 2 | 2 | | |
| POE Sorbitan Monooleate | 3.6 | | | | | | 3.6 | | |
| POE Cetyl Ether | | 0.8 | 0.8 | | | | | | |
| POE Lauryl Ether | | 2 | 2 | | | | | | 0.1 |
| POE Nonylphenyl Ether | | 1 | 1 | | | | | | |
| Propylene Glycol | 2 | | 2 | | | 2 | 2 | | |
| Sodium Pyrrolidone Carboxylate | | 2 | | | | | | | |
| Perfume | Appropriate amount | | | | | | | | |
| Color Additive | Appropriate amount | | | | | | | | |
| Preservative | Appropriate amount | | | | | | | | |
| pH-Adjuster | Appropriate amount | | | | | | | | |
| Ethanol | 53 | 55 | 55 | 80 | 38 | 58 | 58 | 55.1 | 56.5 |
| Water | 10 | 7.9 | 7.9 | 8.1 | 3 | 9.49 | 8.34 | 8.69 | 9.0 |

## EXAMPLES 11-13

| Example No.<br>Components | 11 | 12 | 13 |
|---|---|---|---|
| Castor oil | 8 | 15 | 10 |
| Isostearic acid | 15 | 20 |  |
| Isopalmitic acid |  |  | 15 |
| Isopropyl myristate |  | 3 |  |
| Oleyl alcohol | 7 |  |  |
| Octyldodecanol |  |  | 2.5 |
| Color Additive | Appropriate | | |
| Preservative | Appropriate | | |
| Antioxidant | Appropriate | | |
| Perfume | Appropriate | | |
| Ethanol | 60 | 55 | 65 |
| Water | 5 | 3 | 5 |

The effect of this invention is further explained by the following comparison data between compositons of this invention and commercially available compositions (hereinafter abbreviated as CACs).

EXPERIMENT 1

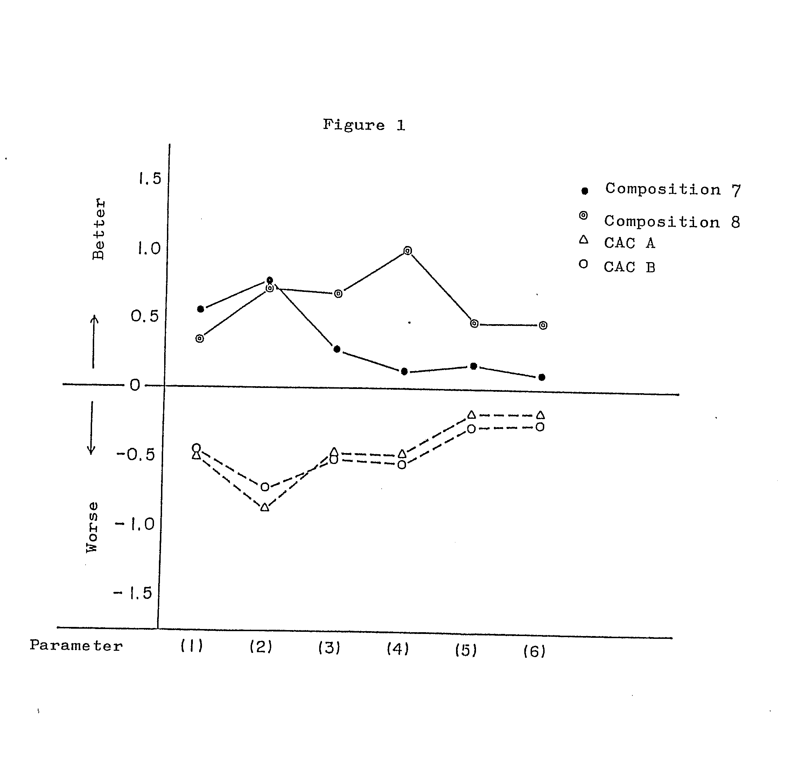

A comparison test between compositions of this invention and commercially available ones was applied to volunteers (120 persons) and evaluated by the one to one comparison method. In the test, two different kinds of compositions are applied to the right and left parts of a volunteer's head at 2 ml each. The evaluation was carried out by a hair-dresser according to the following parameters. The results were totalized according to Sheffe's Method concerning one to one comparison methods (Figure 1).

(Test Compositions)
1.   Composition 7 (●): The composition prepared in Example 7,
2.   Composition 8 (◉): The composition prepared in Example 8,
3.   CAC A (△), and
4.   CAC B (◯).

(Parameters for Evaluation)
1.   Spreadability,
2.   Feeling in rubbing into the hair,
3.   Brushability just after applied,
4.   Manageability of the hair,
5.   Softness after hair-dried, and

6.    Overall evaluation after hair-dressed.


EXPERIMENT 2


In the same manner as in Experiment 1, the following compositions were tested (Figure 1).


(Test Compositions)


1.    Composition 9 (●): The composition prepared in Example 9,

2.    Composition 3 (◉): The composition prepared in Example 3,

3.    Composition 10 (△): The composition prepared in Example 10, and

4.    CAC A (○).


(Results)


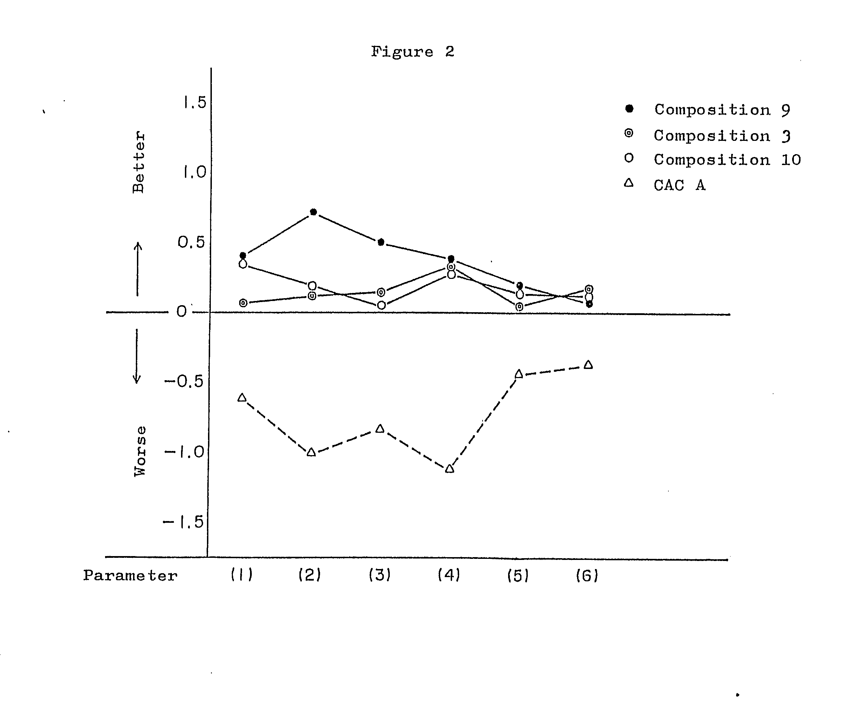

In Figures 1 and 2, the six parameters aforesaid are shown as the ordinate axis and totalized result on each parameter is shown as the abscissa axis:  the results show, on the corresponding parameters, how good the compositions are.  Obviously from the figures, hair lotions of this invention are superior to any CACs in every parameter; this is a good explanation of the characteristics of hair lotions of this invention.


EXPERIMENT 3


Both a composition of this invention and a CAC (about 30 ml each) were applied to several male volunteers and the feel in use was tested between the two according to the one to one comparison method by applying the two types of compositions to the right and left parts of the head, at the same amount of each, for a week. The volunteers applied the composition at an amount that they wanted. One week later, the volunteers reported which they liked better in a questionnaire.

(Parameters for Evaluation)

1.  Manageability of the hair,
2.  Brushability,
3.  Lustre,
4.  Fragrance, and
5.  Overall evaluation.

The results on the overall evaluation are shown in the following Table 1.

Table 1

| Composition Tested | | Number of Volunteers answered "Superior" | | Number of Volunteers |
|---|---|---|---|---|
| | | Invention | CACs | |
| Exam. 4 | CAC C | 8 | 4 | 12 |
| Exam. 8 | CAC D | 6 | 3 | 10 (1)* |
| Exam. 3 | CAC D | 8 | 5 | 14 (1) |
| | CAC A | 9 | 4 | 14 (1) |
| | CAC E | 10 | 4 | 14 |

* Note: In the Table, numerals surrounded by parenthesis show how many persons answered "No difference".

CLAIMS:

1. The use of a branched $C_{12}-C_{22}$ saturated monocarboxylic acid in a solubilizing function in a hair composition.

2. The use claimed in claim 1, wherein the acid is employed in an amount of 5 to 31 w/v %.

3. The use claimed in claim 1 or claim 2, wherein a surfactant is also employed in a small amount.

4. The use claimed in claim 3, wherein the amount of surfactant is up to 5 w/v %.

5. A hair lotion including castor oil, an alcohol, water and, as a solubilizer, a branched $C_{12}-C_{22}$ saturated monocarboxylic acid 5 to 31 w/v % and a surfactant 0 to 5 w/v %.

6. A hair lotion as claimed in claim 5, wherein the branched $C_{12}-C_{22}$ saturated monocarboxylic acid is isostearic acid.

7. A hair lotion as claimed in claim 5 or claim 6, wherein the alcohol is ethanol.

8. A hair lotion consisting essentially of:

| | |
|---|---|
| castor oil | 5-22 w/v % |
| isostearic acid | 5-31 w/v % |
| ethanol | 85-38 v/v % |
| a surfactant | 0-5 w/v %, and |
| water | necessary amount to make 100%. |

9.    A method of improving the cosmetic appearance of hair which comprises applying thereto a hair lotion as claimed in any one of claims 5 to 8 or a hair composition prepared using a branched $C_{12}$-$C_{22}$ saturated monocarboxylic acid in a solubilizing function.

Figure 1

Figure 2